(19) Europäisches Patentamt
European Patent Office
Office européen des brevets

(11) Publication number: **0 051 409**
**B1**

(12) # EUROPEAN PATENT SPECIFICATION

(45) Date of publication of patent specification: **21.03.84**

(51) Int. Cl.³: **C 07 D277/56**, A 61 K 31/425

(21) Application number: **81305012.7**

(22) Date of filing: **23.10.81**

(54) **2-(Substituted-phenyl)-thiazole derivatives suitable for peptic ulcer treatment.**

(30) Priority: **24.10.80 JP 149200/80**

(43) Date of publication of application:
**12.05.82 Bulletin 82/19**

(45) Publication of the grant of the patent:
**21.03.84 Bulletin 84/12**

(84) Designated contracting states:
**DE FR GB IT**

(56) References cited:
**FR-A- 1 546 183**
**FR-A- 2 152 056**
**GB-A- 2 020 661**
**GB-A- 2 020 662**
**GB-A- 2 056 440**
**PATENTS ABSTRACTS OF JAPAN, vol. 5, no. 120, August 4, 1981, (C65) (792)**

(73) Proprietor: **KUREHA KAGAKU KOGYO KABUSHIKI KAISHA**
**9-11 Horidome-cho 1-chome Nihonbashi Chuo-ku**
**Tokyo (JP)**

(72) Inventor: **Kawasaki, Takao**
**532-27 Mizuno**
**Sayama-shi Saitama-ken (JP)**
Inventor: **Immaru, Daisaku**
**104 5-1-3 Toyogaoka**
**Tama-shi Tokyo (JP)**
Inventor: **Tsuchiya, Tadashi**
**3-1119 Matsudo**
**Matsudo-shi Chiba-ken (JP)**
Inventor: **Yamaguchi, Yukiharu**
**203 1-13-1 Tamazutsumi**
**Setagaya-ku Tokyo (JP)**
Inventor: **Komatsu, Katsumi**
**3-1119 Matsudo**
**Matsudo-shi Chiba-ken (JP)**

(74) Representative: **Myerscough, Philip Boyd et al**
**J.A.Kemp & Co. 14, South Square Gray's Inn**
**London, WC1R 5EU (GB)**

**EP 0 051 409 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

2-(substituted-phenyl)-thiazole derivatives suitable for peptic ulcer treatment

The present invention concerns 2-(substituted-phenyl)-thiazoles which are useful as anti-peptic ulcer medicines, their preparation and pharmaceutical compositions containing them.

Peptic ulcers are collapsed, weakened parts of the gastric or enteric mucosa caused by the action of aggressive factors such as hydrochloric acid and pepsin in the gastric juice. Mild cases of peptic ulcer are curable after 3 to 4 months of hospitalization and treatment. However, serious cases are accompanied by a haemorrhaging and perforation of the organ which cause the problem to become chronic.

Abnormalities in the automatic nerve system and in the mucosal blood flow due to physical and/or mental stress have been considered as etiological causes of peptic ulcers. However, it is practically impossible to interpret the etiology of peptic ulcers unitarily because the viscera themselves are subjected to complicated control by nerves and hormones.

Sodium hydrogen carbonate, aluminium salts and magnesium salts have been used for a long time as antipeptic ulcer medicines as a means of neutralizing the acid in the gastric juice. However, these medicines only temporarily neutralize the acid to alleviate the pain and do not accelerate a substantial cure of the ulcer.

Recently, many kinds of anti-ulcer medicines have been developed based on presumed causes of ulcers, including medicines for suppression of the automatic nerve, that is, so-called anti-cholinergic agents, agents for repairing the damaged tissues and agents for improving the blood flow. However, the present situation is that none of them can be said to be satisfactory in view of their ineffectiveness or their side effects.

For instance, carbenoxolone which has been commercialized as an anti-peptic ulcer medicine has been broadly used because of its excellent effect in accelerating the cure of ulcers. However, it has aldosterone-like side effects which cause hypertension and weakening of muscular functions when it is taken continuously. The anti-cholinergic agents show severe side effects such as mydriasis and thirst due to the blocking of the parasympathetic nerve, and it has been reported that their effects of accelerating the cure of ulcers is low.

Since it generally takes a long time to cure a peptic ulcer, the period of administration of an antipeptic ulcer medicine extends from 100 to 150 days on average. Accordingly, it is required that an anti-peptic ulcer medicine should be very safe as well as highly effective in curing ulcers.

Compounds have now been found which can exhibit an excellent anti-peptic ulcer action and are pharmacologically safe. The compounds according to the present invention are 2-(substituted-phenyl)-thiazole derivatives represented by the following formula :

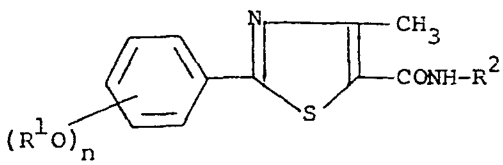

(I)

wherein $R^1$ is an alkyl group of from 1 to 3 carbon atoms, $R^2$ is a hydrogen atom or an alkyl group of from 1 to 3 carbon atoms and n is an integer of from 1 to 3, with the proviso that when $R^2$ represents a hydrogen atom the 3-, 4- and 5- positions of the benzene ring are not simultaneously occupied by methoxy groups.

2-(Substituted-phenyl)-thiazole derivatives represented by formula (I) have excellent anti-peptic ulcer action and are pharmacologically safe. 2-(Substituted-phenyl)-thiazole derivatives according to the present invention (hereinafter referred to as the present compounds) include the compounds shown in Table 1 below. The melting point, appearance and elementary analytical composition of the compounds are also shown.

Table 1

| Compound Number | Name of compound | Structural formula | Melting point (°C) |
|---|---|---|---|
| 1 | 2-(3,4,5-trimethoxyphenyl)-4-methylthiazole-5-methylcarboxamide | 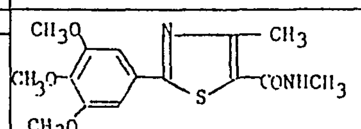 | 177.5 - 179.5 |
| 2 | 2-(3,4,5-trimethoxyphenyl)-4-methylthiazole-5-n-propylcarboxamide | 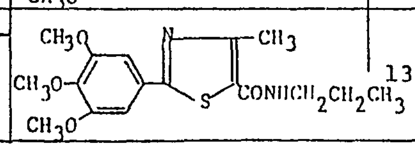 | 132 - 133 |

Table 1 (Continued)

| Compound Number | Name of compound | Structural formula | Melting point (°C) |
|---|---|---|---|
| 3 | 2-(3,4-dimethoxyphenyl)-4-methylthiazole-5-carboxamide | 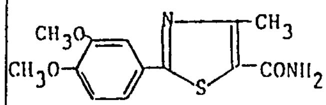 | 218 - 219.5 |
| 4 | 2-(3,4-dimethoxyphenyl)-4-methylthiazole-5-methylcarboxamide | 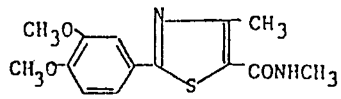 | 181.5 - 183 |
| 5 | 2-(3,4-dimethoxyphenyl)-4-methylthiazole-5-n-propylcarboxamide | 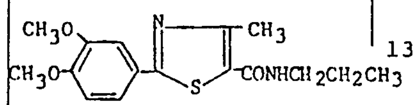 | 135 - 136 |
| 6 | 2-(2-methoxyphenyl)-4-methylthiazole-5-carboxamide | 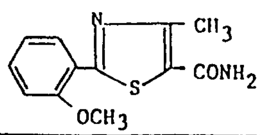 | 191 - 192.5 |

Table 1 (Continued)

| Compound Number | Name of compound | Appearance | Elementary analytical composition (%) C | H | N | S |
|---|---|---|---|---|---|---|
| 1 | 2-(3,4,5-trimethoxyphenyl)-4-methylthiazole-5-methylcarboxamide | colourless minute aciculate | 55.91 (55.89) | 5.63 (5.63) | 8.70 (8.69) | 9.90 (9.94) |
| 2 | 2-(3,4,5-trimethoxyphenyl)-4-methylthiazole-5-n-propylcarboxamide | colourless minute aciculate | 58.25 (58.27) | 6.30 (6.32) | 7.99 (7.99) | 9.17 (9.15) |
| 3 | 2-(3,4-dimethoxyphenyl)-4-methylthiazole-5-carboxamide | colourless minute aciculate | 56.11 (56.08) | 5.06 (5.07) | 10.09 (10.06) | 11.55 (11.51) |
| 4 | 2-(3,4-dimethoxyphenyl)-4-methylthiazole-5-methylcarboxamide | colourless aciculate | 57.50 (57.52) | 5.55 (5.52) | 9.58 (9.58) | 10.94 (10.97) |
| 5 | 2-(3,4-dimethoxyphenyl)-4-methylthiazole-5-n-propylcarboxamide | colourless flake | 59.94 (59.98) | 6.32 (6.29) | 8.72 (8.74) | 10.05 (10.01) |
| 6 | 2-(2-methoxyphenyl)-4-methylthiazole-5-carboxamide | colourless minute aciculate | 58.04 (58.05) | 4.88 (4.87) | 11.28 (11.28) | 12.88 (12.91) |

Table 1 (Continued)

| Com-Pound No | Name of Compound | Structural formula | Melting Point (°C) |
|---|---|---|---|
| 7 | 2-(2-methoxyphenyl)-4-methylthiazole-5-methyl-carboxamide | 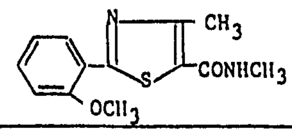 | 167.5 - 168.5 |
| 8 | 2-(2-methoxyphenyl)-4-methylthiazole-5-n-propylcarboxamide | 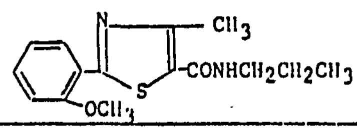 | 117.5 - 119 |
| 9 | 2-(4-methoxyphenyl)-4-methylthiazole-5-carboxyamide | 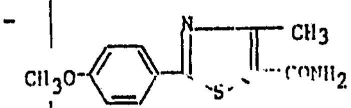 | 179.5 - 180.5 |
| 10 | 2-(4-methoxyphenyl)-4-methylthiazole-5-methylcarboxamide | 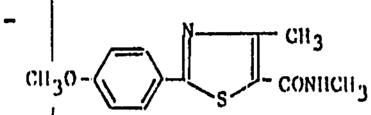 | 157.5 - 159 |
| 11 | 2-(4-methoxyphenyl)-4-methylthiazole-5-n-propylcarboxamide | 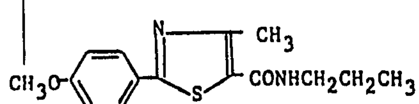 | 126 - 126.5 |

Table 1 (Continued)

| Com-Pound No | Name of Compound | Appearance | Elementary analytical composition (%) C | H | N | S |
|---|---|---|---|---|---|---|
| 7 | 2-(2-methoxyphenyl)-4-methylthiazole-5-methyl-carboxamide | colourless aciculate | 59.49 (59.52) | 5.41 (5.38) | 10.66 (10.68) | 12.19 (12.22) |
| 8 | 2-(2-methoxyphenyl)-4-methylthiazole-5-n-propylcarboxamide | colourless plate | 62.01 (62.02) | 6.27 (6.25) | 9.66 (9.64) | 11.04 (11.04) |
| 9 | 2-(4-methoxyphenyl)-4-methylthiazole-5-carboxyamide | colourless aciculate | 58.09 (58.05) | 4.85 (4.87) | 11.27 (11.28) | 12.88 (12.91) |
| 10 | 2-(4-methoxyphenyl)-4-methylthiazole-5-methylcarboxamide | colourless minute aciculate | 59.55 (59.52) | 5.36 (5.38) | 10.65 (10.68) | 12.18 (12.22) |
| 11 | 2-(4-methoxyphenyl)-4-methylthiazole-5-n-propylcarboxamide | slightly yellow flake | 62.00 (62.02) | 6.27 (6.25) | 9.65 (9.64) | 11.01 (11.04) |

Note : The parenthesized values in parenthesis in the Elementary Analytical Composition column represent theoretical values based on the molecular formula of each compound.

The present compounds can be synthesized by effecting direct ammonolysis of an alkyl ester of a 2-(substituted-phenyl)-4-methylthiazole-5-carboxylic acid represented by the general formula (II) :

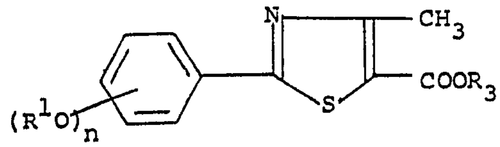

(II)

wherein $R^1$ and $R^3$ independently represent an alkyl group of from 1 to 3 carbon atoms and n denotes an integer of from 1 to 3. The reaction can be represented by :

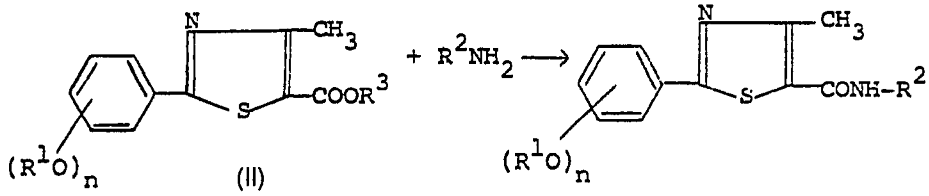

wherein $R^1$ and $R^3$ have the same meanings as above, $R^2$ represents a hydrogen atom or an alkyl group of from 1 to 3 carbon atoms and n denotes an integer of 1 to 3. It will be appreciated that the term « ammonolysis » is used broadly to mean reaction of a compound of formula (II) with an amide of formula $R^2NH_2$ in which $R^2$ denotes an alkyl group of from 1 to 3 carbon atoms as well as with ammonia itself. The reaction is carried out in a single solvent such as alcohol, or in a mixed solvent such as an aqueous ammonia and alcohol. Reaction can be effected at a temperature of from – 30 to 150 °C, preferably from 0 to 100 °C, for from 0.1 hour to 15 days. The reaction product can then be recrystallized from an aqueous alcoholic solution.

The alkyl ester of formula (II) used as the starting material can be obtained by refluxing together a substituted benzoic acid thioamide and an alphahaloketone in the presence of phosphorus pentasulfide and in an organic solvent such as benzene, toluene, xylene, methylchloroform or ethanol, according to the following reaction formula :

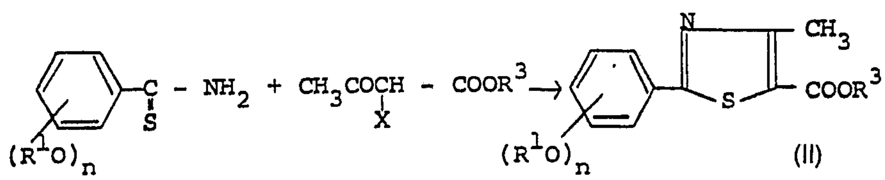

wherein $R^1$, $R^3$ and n have the same meanings as above, and X represents a halogen atom.

The important problem in the development of an anti-peptic ulcer medicine is the screening of the medicine. Hitherto, the evaluation of anti-ulcer medicines has been frequently carried out based on their prophylactic effect against acute ulcers such as ulcers due to pyloric ligation, aspirin or indomethacin. However, to what extent the results of evaluations by these models reflect the effect of the medicines on human ulcers has not been fully elucidated.

The present inventors, taking into account this situation, included with the above-mentioned evaluation methods a technique based on the oral administration of the present compound and a commercial anti-peptic ulcer medicine, respectively, to rats in which duodenal peptic ulcers due to acetic acid (refer to Okabe, 1971), considered to most closely resemble human peptic ulcers, had been artificially induced.

Anti-peptic ulcer effect of the present compound :

(1) Effect on peptic ulcers due to pyloric ligation

Thirteen groups (10 animals per group) of male rats weighing 180 to 200 g were subjected to ligation of their pylori under ether-anesthesia after fasting for 48 hours according to the method of Shay *et al.* (Refer to Gastroenterology, *5,* page 43 (1945)).

Just after ligation, one of the present compounds suspended in an aqueous physiological saline solution was intraperitoneally administered to a rat. Into the rats of one control group an aqueous physiological saline solution was injected, whilst into rats of another control group (a positive control group) Gefarnate was injected. Then, after 15 hours of fasting without water, the rats were sacrificed with ether. Their stomachs were removed for examination under a microscope. The length and width of the thus-formed ulcers in each stomach were determined and their product calculated ($mm^2$). The total sum

of the products represented the ulcer coefficient. The results are shown in Table 2. As shown in Table 2, the ulcer coefficients of the present compounds are much better than that of Gefarnate.

Table 2

| Compound number | Dose rate (mg/kg) | Ulcer coefficient ($mm^2$) | Rate of suppression[1)] (%) |
|---|---|---|---|
| 1 | 100 | 14.7 | 65.0 |
| 2 | 100 | 6.3 | 85.0 |
| 3 | 100 | 0.8 | 98.1 |
| 4 | 100 | 11.0 | 73.8 |
| 5 | 100 | 8.4 | 80.0 |
| 6 | 100 | 1.2 | 97.1 |
| 7 | 100 | 8.6 | 79.5 |
| 8 | 100 | 9.0 | 78.6 |
| 9 | 100 | 0.9 | 97.9 |
| 10 | 100 | 6.4 | 84.8 |
| 11 | 100 | 8.5 | 79.8 |
| Positive control[2)] | 100 | 36.9 | 12.1 |
| Control | - | 42.0 | 0 |

Notes

1) Rate of suppression of ulcer = (Ulcer coefficient (control)-Ulcer coefficient (treated group)/Ulcer coefficient (control)) × 100

2) Positive control : Gefarnate = 3,7-dimethyl-2,6-octadienyl 5,9,13-trimethyl-4,8,12-tetradecatrienoate

(2) Effect on peptic ulcers due to acetic acid

Following the method of Okabe *et al* (refer to Amer. J. Dig. Dis., *16,* page 277 (1977)), 13 groups (15 animals per group) of male rats weighing 240 to 260 g were subjected to laparotomy under ether anesthesia in which a metal circular frame was placed on the serosa at a distance of 5 to 7 mm from the duodenal pylorus, and 0.06 ml of glacial acetic acid was poured into the frame. After 30 seconds, the liquid containing the acetic acid was removed and then the frame was removed. One test compound suspended in an aqueous physiological saline solution was orally administered to a rat 3 times a day from the third day after the operation for 10 consecutive days. Into the rats of one control group, only an aqueous physiological saline solution was administered whilst Gefarnate (a positive control) was injected into the rats of another control group. After administration, the rats were sacrificed with ether, and their duodenum were removed for observation under a microscope. The length and width of the thus-formed ulcers were measured and their product (expressed in $mm^2$) was recorded as the ulcer coefficient. The results are shown in Table 3. As shown in Table 3, the ulcer coefficients of the present compounds are much better than that of Gefarnate.

Table 3

| Compound number | Dose rate (mg/kg) | Ulcer coefficient ($mm^2$) | Rate of suppression of ulcer (%) |
|---|---|---|---|
| 1 | 100 | 3.1 | 65.6 |
| 2 | 100 | 2.7 | 70.0 |
| 3 | 100 | 1.3 | 85.6 |
| 4 | 100 | 1.8 | 80.0 |
| 5 | 100 | 2.9 | 67.8 |
| 6 | 100 | 2.1 | 76.7 |

(Continued)

| Compound number | Dose rate (mg/kg) | Ulcer coefficient (mm²) | Rate of suppression of ulcer (%) |
|---|---|---|---|
| 7 | 100 | 1.7 | 81.1 |
| 8 | 100 | 3.2 | 64.4 |
| 9 | 100 | 2.0 | 77.8 |
| 10 | 100 | 3.1 | 65.6 |
| 11 | 100 | 2.5 | 72.2 |
| Positive control 1) | 100 | 7.2 | 20.0 |
| Control | - | 9.0 | 0 |

Note : 1) Positive control : Gefarnate (refer to the footnote of Table 2)

Using this method of evaluation, effectiveness is not seen with antiacid and anticholinergic medicines, both of which have been conventionally used as anti-ulcer medicines, and only a slight effectiveness is recognised in respect of Gefarnate which is intended to repair damaged tissues. On the other hand, in the groups of rats to which one of the present compounds was administered, a remarkable curative effect was recognised. Even on histological observation of the ulcer lesions, a state of complete cure has been obtained.

This experimental model has been highly evaluated internationally because the thus-formed ulcer is scarcely curable in nature and the histopathological change of the ulcer lesions closely resembles that of human chronic ulcer as compared to the method of cautery-ulcer (refer to Skoryna, 1958) and the method of crumping-cortisone (refer to Umehara, 1965).

(3) On the evaluation by the hitherto broadly utilized effective methods for screening anti-peptic ulcer medicines such as those based on stress, aspirin and indomethacin-induced ulcers, the present compounds showed superior effects to the effects of commercial anti-peptic ulcer medicines.

Toxicological properties of the present compound :

(1) Acute toxicity test

Experimental animals

Female ICR-mice of body weight of 20 to 24 g, 5 weeks after birth were used.

Method of rearing :

Ten animals per group were kept in a transparent polycage at room temperature of 23 ± 1 °C, and RH of 60 to 70 %.

Administration of the present compound :

After minutely pulverizing each one of the present compounds, a pulverized compound was suspended in an aqueous 5 % sodium carboxymethyl-cellulose solution containing 20 % of Tween-80. The aqueous suspension was forcibly orally administered by a metal stomach tube, the dose rate having been adjusted by changing the concentration of the present compound in the aqueous suspension.

General symptoms due to administration of the present compound :

In cases of administration at higher dose rates, the rats became inactive but, after 2 to 3 hours, they became normal. In some cases, where death occurred, spontaneous movement was reduced with reduction of general tension and the rats died as they were.

Calculation of $LD_{50}$ :

The rats' mortality was observed for a week after administration. The $LD_{50}$ was calculated from the mortality rate by Litchfield-Wilcoxon's formula. The results are shown in Table 4. As shown in Table 4, the present compounds have a very high safety.

Table 4

| Compound Number | $LD_{50}$ (p.o.)(mg/kg) |
|---|---|
| 1 | 5200 |
| 2 | 5600 |
| 3 | 6200 |
| 4 - 11 | more than 8000 |

In addition, according to the results of acute toxicity tests using rats and mice as experimental animals, the $LD_{50}$ i. v. was larger than 1.2 g/kg.

(2) Sub-acute toxicity test

Experimental animals :

Both sexes of Sprague-Dowley rats of 110 to 150 g body weight 5 months after their birth were used.

Method of rearing :

Each five males and five females respectively were kept in a metal wire-net cage at room temperature of 22 to 24 °C and RH of 60 to 70 % for 3 months, each experimental group consisting of 10 males or 10 females.

Administration of the present compound :

Compound No. 3 of the present compounds, 2-(3,4-dimethoxyphenyl)-4-methylthiazole-5-carboxamide, was minutely pulverized and mixed with the powdery diet of the rat at a concentration of 0.4 % by weight. The thus prepared diet was taken *ad lib.* The mean intake of the present compound was 400 mg/kg/day.

Examination :

The diet intake and the body weight of each rat were measured every other day and once a week, respectively. Urinalysis for glucose, protein, pH and occult blood was carried out once a month. Blood samples were examined after ending the rearing. After sacrificing all the animals, they were autopsied to examine for the presence of abnormalities. Their organs were fixed with formaldehyde and embedded in paraffin to prepare sliced specimens of tissues stained with hematoxylineosine for microscopic observation.

Results :

(a) Diet intake was normal without a significant difference between experimental groups and the control group.
(b) Body weight gain was normal without a significant difference between experimental groups and the control group.
(c) Mortality, (d) urinalysis, (e) hematological examination, and (f) findings on autopsy and histological examination were all normal without any significant difference between experimental groups and the control group.

Further, in sub-acute toxicity tests using mice as experimental animals, abnormal findings attributable to the present compounds were never obtained.

As is seen above, the present compound is very safe for administration. Accordingly, it can be used as an anti-peptic ulcer medicine for humans.

In addition to its excellemt pharmacological effects and toxicological properties, every compound of the present invention is colourless and crystalline, and almost all of them are tasteless or are only slightly bitter. Furthermore, since they are extremely stable and can be stored without any change at room temperature in an open state, their adaptability as an anti-peptic ulcer medicine can be said to be remarkably high.

The present invention provides a pharmaceutical composition comprising a compound of formula (I) as active ingredient, together with a pharmaceutically acceptable carrier.

Pharmaceutical compositions according to the present invention are useful for treatment of peptic

ulcers, and can comprise in unit dosage form a therapeutically effective amount of a compound of formula (I) together with a pharmaceutically acceptable carrier. The pharmaceutical composition in unit dosage form can be, e. g. tablets, sugar-coated tablets, pills, capsules, powders, granules, troches, liquids, suppositories, injections, etc.

As the carrier, lactose, sucrose, sorbitol, mannitol, potato-starch, corn-starch, amylopectin, various kinds of starch, derivatives of cellulose (for instance carboxymethylcellulose and methylcellulose), gelatin, magnesium stearate, calcium stearate, polyvinyl alcohol, polyethylene glycol waxes, gum arabic, talc, titanium dioxide, vegetable oils such as olive oil, peanut oil and sesame oil, paraffin oil, neutral fatty bases, ethanol, aqueous physiological saline solutions, sterilized water, glycerol, colouring agents, flavorings, thickening agents, stabilizers, isotonic agents and buffering agent can be exemplified.

The content of the one of the present compounds in a pharmaceutical composition according to the invention is preferably 0.1 to 90 % by weight, more preferably 1 to 60 % by weight of the preparation.

The clinical daily dose of the present compound can be 60 to 6,000 mg/60 kg of body weight, preferably 150 to 3,000 mg/60 kg body weight. The route of administration may be oral or by injection, preferably orally in the case of long term administration.

The present compound does also exhibit various other activities such as gastric acid-secretoinhibitory activity, dilative activities on peripheral veins and the bronchus, hypotensive activity, antiarrhythmic activity and anti-inflammatory activity.

The following Examples illustrate the present invention. Throughout the description percentages are by weight unless otherwise specified.

## Synthetic Examples

### Example 1

Synthesis of 2-(3,4,5-trimethoxyphenyl)-4-methylthiazole-5-methylcarboxamide

16.9 Grams of ethyl 2-(3,4,5-trimethoxyphenyl)-4-methylthiazole-5-carboxylate, which had been prepared by heating a mixture of 3,4,5-trimethoxybenzamide, phosphorus pentasulfide, ethyl α-chloroacetoacetate and n-butylalcohol under a reflux condenser for 5 hours, was dissolved in a mixture of 80 ml of a 40 % solution of methylamine and 420 ml of ethanol. The solution was left for 10 days at room temperature. Then the solution was condensed to a solid under a reduced pressure. The thus obtained residue was recrystallized from an aqueous ethanolic solution to obtain the desired compound as colourless minute aciculates melting at 177.5 to 179.5 °C in an amount of 10.5 g, corresponding to a yield of 60 %.

### Example 2

Synthesis of 2-(3,4,5-trimethoxyphenyl)-4-methylthiazole-5-propylcarboxamide

A mixture of 8.5 g of ethyl 2-(3,4,5-trimethoxy-phenyl)-4-methylthiazole-5-carboxylate, 2.3 g of n-propylamine and 100 ml of ethanol was put into an ampoule. After sealing, the ampoule was kept at 60 °C for 8 hours. After opening the ampoule and condensing the content to a solid, the residue was recrystallized from an aqueous ethanolic solution to obtain 5.8 g of the desired compound as colourless minute aciculates melting at 132 to 133 °C, corresponding to a yield of 61 %.

### Example 3

Synthesis of 2-(3,4-dimethoxyphenyl)-4-methylthiazole-5-carboxamide

15.4 Grams of ethyl 2-(3,4-dimethoxyphenyl)-4-methylthiazole-5-carboxylate, which had been prepared by heating a mixture of 3,4-dimethoxybenzamide, ethyl α-chloroacetoacetate and n-butyl alcohol under reflux for 5 hours, was dissolved in a mixed solution of 50 ml aqueous 28 % ammonia and 450 ml of ethanol. This solution was left at room temperature for 7 days. After condensing the solution to a solid under reduced pressure, the thus obtained residue was recrystallized from an aqueous ethanolic solution to obtain the desired compound as colourless minute aciculates melting at 218 to 219.5 °C in an amount of 9.0 g, corresponding to a yield of 65 %.

### Example 4

Synthesis of 2-(3,4-dimethoxyphenyl)-4-methylthiazole-5-methylcarboxamide

15.4 Grams of ethyl 2-(3,4-dimethoxyphenyl)-4-methylthiazole-5-carboxylate was dissolved in a mixed solvent of 80 ml of 40 % methylamine and 600 ml of ethanol. Then, in a similar manner to Example 1, the desired compound is obtained as colourless aciculates melting at 181.5 to 183 °C in an amount of 9.6 g, corresponding to a yield of 66 %.

## Example 5

Synthesis of 2-(3,4-dimethoxyphenyl)-4-methylthiazole-5-propylcarboxamide

A mixture of 15.4 g of ethyl 2-(3,4-dimethoxy-phenyl)-4-methylthiazole-5-carboxylate, 5 g of n-propylamine and 300 ml of ethanol was kept at 50 °C for 2 hours and left for a further 3 days at room temperature. Then, the thus formed solution was condensed to a solid under a reduced pressure. The residue was recrystallized from an aqueous ethanolic solution to obtain the desired compound as colourless flakes melting at 135 to 136 °C in an amount of 10.0 g, corresponding to a yield of 63 %.

## Example 6

Synthesis of 2-(2-methoxyphenyl)-4-methylthiazole-5-carboxamide

13.9 Grams of ethyl 2-(2-methoxyphenyl)-4-methylthiazole-5-carboxylate, which had been prepared by heating a mixture of o-methoxybenzamide, ethyl α-chloroacetoacetate and phosphorus pentasulfide under a reflux condenser for 5 hours, was dissolved in a solvent mixture of 50 ml of aqueous 28 % ammonia and 450 ml of ethanol. Then in a similar manner to Example 3, the desired compound was obtained as colourless minute aciculates melting at 191 to 192.5 °C in an amount of 8.7 g, corresponding to a yield of 70 %.

## Example 7

Synthesis of 2-(2-methoxyphenyl)-4-methylthiazole-5-methylcarboxamide

13.9 Grams of ethyl 2-(2-methoxyphenyl)-4-methylthiazole-5-carboxylate was dissolved in a mixed solvent of 80 ml of 40 % methylamine and 600 ml of ethanol. Then, in a similar manner to Example 1, the desired compound was obtained as colourless aciculates melting at 167.5 to 168.5 °C in an amount of 9.4 g, corresponding to a yield of 72 %.

## Example 8

Synthesis of 2-(2-methoxyphenyl)-4-methylthiazole-5-propylcarboxamide

13.9 Grams of ethyl 2-(2-methoxyphenyl)-4-methylthiazole-5-carboxylate was dissolved in a mixed solvent of 5 g of n-propylamine and 300 ml of ethanol. Then, in a similar manner to Example 6, the desired compound was obtained as colourless plates melting at 117.5 to 119 °C in an amount of 8.7 g, corresponding to a yield of 60 %.

## Example 9

Synthesis of 2-(4-methoxyphenyl)-4-methylthiazole-5-carboxamide

13.9 Grams of ethyl 2-(4-methoxyphenyl)-4-methylthiazole-5-carboxylate, which had been prepared by heating a mixture of p-methoxybenzamide, ethyl α-chloroacetoacetate, phosphorus pentasulfide and n-butyl alcohol under reflux for 5 hours, was dissolved in a mixed solvent of 50 ml of aqueous 28 % ammonia and 450 ml of ethanol. Then, in a similar manner to Example 3, the desired compound was obtained as colourless aciculates melting at 179.5 to 180.5 °C in an amount of 8.6 g, corresponding to a yield of 69 %.

## Example 10

Synthesis of 2-(4-methoxyphenyl)-4-methylthiazole-5-methylcarboxamide

13.9 Grams of ethyl 2-(4-methoxyphenyl)-4-methylthiazole-5-carboxylate was dissolved in a mixed solvent of 80 ml of 40 % methylamine and 600 ml of ethanol. Then, in a similar manner to Example 1, the desired compound was obtained as colourless minute aciculates melting at 157.5 to 159 °C in an amount of 9.1 g, corresponding to a yield of 69 %.

## Example 11

Synthesis of 2-(4-methoxyphenyl)-4-methylthiazole-5-propylcarboxamide

13.9 Grams of ethyl 2-(4-methoxyphenyl)-4-methylthiazole-5-carboxylate was dissolved in a mixed solvent of 5 g of n-propylamine and 300 ml of ethanol. Then, in a similar manner to Example 6, the desired

compound was obtained as slightly yellow flakes melting at 126 to 126.5 °C in an amount of 9.7 g, corresponding to a yield of 67 %.

Pharmaceutical compositions

Example 12

Manufacture of the granular preparation for oral administration :

Two hundred grams of 2-(3,4-dimethoxyphenyl)-4-methylthiazole-5-carboxamide was minutely pulverized. 800 Grams of corn-starch was admixed with the pulverized compound. After stirring the mixture well, 80 ml of an aqueous solution containing 3 g of sodium carboxymethylcellulose dissolved therein was added to the mixture. After kneading the whole mixture, it was subjected to an extruding pelletizer to form granules. These granules were dried at a temperature of 60 to 80 °C and screened to obtain the granular preparation for oral administration.

**Claims**

1. A compound which is a 2-(substituted-phenyl)-thiazole having the formula (I) :

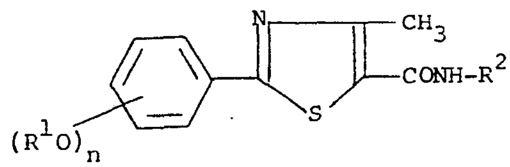

(I)

wherein $R^1$ represents an alkyl group of from 1 to 3 carbon atoms, $R^2$ represents a hydrogen atom or an alkyl group of from 1 to 3 carbon atoms and n denotes an integer of from 1 to 3, with the proviso that when $R^2$ represents a hydrogen atom the 3-, 4- and 5-positions of the benzene ring are not simultaneously occupied by methoxy groups.

2. A compound according to claim 1, wherein $R^1$ is a methyl group.

3. A compound according to claim 1, which is selected from 2-(3,4,5-trimethoxyphenyl)-4-methylthiazole-5-methylcarboxamide and 2-(3,4,5-trimethoxyphenyl)-4-methylthiazole-5-n-propylcarboxamide.

4. A compound according to claim 1, which is selected from 2-(3,4-dimethoxyphenyl)-4-methylthiazole-5-carboxamide, 2-(3,4-dimethoxyphenyl)-4-methylthiazole-5-methylcarboxamide, and 2-(3,4-dimethoxyphenyl)-4-methylthiazole-5-n-propylcarboxamide.

5. A compound according to claim 1, which is selected from 2-(2-methoxyphenyl)-4-methylthiazole-5-carboxamide, 2-(2-methoxyphenyl)-4-methylthiazole-5-methylcarboxamide, 2-(2-methoxyphenyl)-4-methylthiazole-5-n-propylcarboxamide, 2-(4-methoxyphenyl)-4-methylthiazole-5-carboxamide, 2-(4-methoxyphenyl)-4-methythiazole-5-methylcarboxamide, and 2-(4-methoxyphenyl)-4-methylthiazole-5-n-propylcarboxamide.

6. A process for producing a compound of formula (I) as defined in claim 1, which process comprises effecting ammonolysis of an alkyl ester of a 2-(substituted phenyl)-4-methylthiazole-5-carboxylic acid, the ester being represented by the formula (II) :

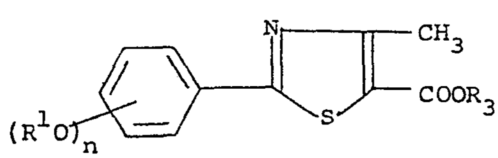

(II)

wherein $R^1$ and n are as defined in claim 1 and $R^3$ is an alkyl group of from 1 to 3 carbon atoms.

7. A process according to claim 6, wherein the reaction is carried out at a temperature of from − 30 to 150 °C.

8. A process according to claim 6 or 7, wherein the ester of formula (II) has been obtained by refluxing together a substituted benzoic acid thioamide of the formula :

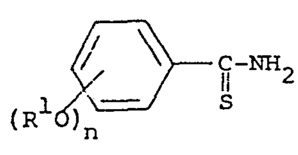

wherein $R^1$ and n are as defined in claim 1, and an alphahaloketone of the formula :

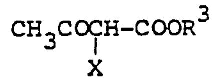

wherein $R^3$ is as defined in claim 6, in the presence of phosphorus pentasulfide and in an organic solvent.

9. A pharmaceutical composition comprising as active ingredient a compound of formula (I) as claimed in any one of claims 1 to 5 or which has been produced by a process as claimed in any of claims 6 to 8, together with a pharmaceutically acceptable carrier.

10. A pharmaceutical composition suitable for treatment of peptic ulcers, which composition comprises in unit dosage form a therapeutically effective amount of a compound of formula (I) as defined in claim 1, and a pharmaceutically acceptable carrier.

## Ansprüche

1. Eine Verbindung, die ein 2-(Phenyl-substituiertes)-thiazol darstellt, der Formel (I) :

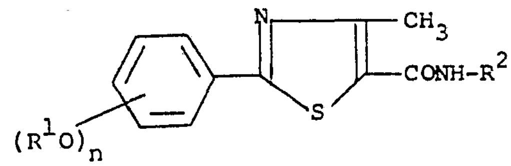

(I)

worin $R^1$ eine Alkylgruppe mit 1 bis 3 Kohlenstoffatomen, $R^2$ ein Wasserstoffatom oder eine Alkylgruppe mit 1 bis 3 Kohlenstoffatomen darstellt und n eine ganze Zahl von 1 bis 3 bedeutet, mit der Maßgabe, daß, wenn $R^2$ ein Wasserstoffatom darstellt, die 3-, 4- und 5-Positionen des Benzolringes nicht gleichzeitig von Methoxygruppen besetzt sind.

2. Verbindung nach Anspruch 1, dadurch gekennzeichnet, daß $R^1$ eine Methylgruppe ist.

3. Verbindung nach Anspruch 1, dadurch gekennzeichnet, daß sie ein 2-(3,4,5-Trimethoyphenyl)-4-methylthiazol-5-methylcarboxamid oder ein 2-(3,4,5-Trimethoxyphenyl)-4-methylthiazol-5-n-propylcarboxamid ist.

4. Verbindung nach Anspruch 1, dadurch gekennzeichnet, daß sie ein 2-(3,4-Dimethoxyphenyl)-4-methylthiazol-5-carboxamid, 2-(3,4-Dimethoxyphenyl)-4-methylthiazol-5-methylcarboxamid oder ein 2-(3,4-Dimethoxyphenyl)-4-methylthiazol-5-n-propylcarboxamid ist.

5. Verbindung nach Anspruch 1, dadurch gekennzeichnet, daß sie ein 2-(2-Methoxyphenyl)-4-methylthiazol-5-carboxamid, 2-(2-Methoxyphenyl)-4-methylthiazol-5-methylcarboxamid, 2-(2-Methoxyphenyl)-4-methylthiazol-5-n-propylcarboxamid, 2-(4-Methoxyphenyl)-4-methylthiazol-5-carboxamid, 2-(4-Methoxyphenyl)-4-methylthiazol-5-methylcarboxamid oder ein 2-(4-Methoxyphenyl)-4-methylthiazol-5-n-propylcarboxamid ist.

6. Verfahren zur Herstellung einer Verbindung der Formel (I) gemäß Anspruch 1, dadurch gekennzeichnet, daß ein Alkylester einer 2-(Phenyl-substituierten)-4-methylthiazol-5-carboxylsäure einer Ammonolyse unterworfen wird, wobei der Ester durch die Formel (II) dargestellt wird :

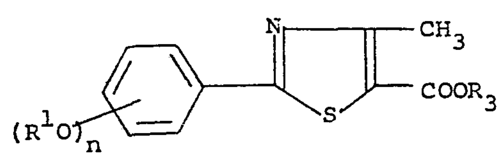

(II)

worin $R^1$ und n die im Anspruch 1 angegebene Bedeutung haben und $R^3$ eine Alkylgruppe mit 1 bis 3 Kohlenstoffatomen ist.

7. Verfahren nach Anspruch 6, dadurch gekennzeichnet, daß die Reaktion bei einer Temperatur von – 30 bis 150 °C durchgeführt wird.

8. Verfahren nach Anspruch 6 oder 7, dadurch gekennzeichnet, daß der Ester der Formel (II) dadurch erhalten wurde, daß ein substituiertes Benzoesäurethiamid der Formel :

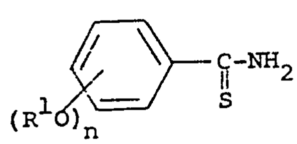

worin $R^1$ und n die im Anspruch 1 angegebene Bedeutung haben, zusammen mit einem alpha-Haloketon der Formel :

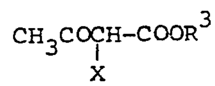

worin $R^3$ die im Anspruch 6 angegebene Bedeutung hat, in Gegenwart von Phosphorpentasulfid und in einem organischen Lösungsmittel unter dem Rückflußkühler behandelt werden.

9. Eine pharmazeutische Zusammensetzung, die als Wirksubstanz eine Verbindung der Formel (I) gemäß einem der Ansprüche 1 bis 5 enthält oder die nach einem Verfahren gemäß einem der Ansprüche 6 bis 8 erhalten wurde, sowie eine pharmazeutisch verträgliche Trägersubstanz.

10. Eine pharmazeutische Zusammensetzung für die Behandlung von Geschwüren, dadurch gekennzeichnet, daß sie als Dosierungseinheit eine therapeutisch wirksame Menge einer Verbindung der Formel (I) gemäß Anspruch 1 und eine pharmazeutisch verträgliche Trägersubstanz enthält.

**Revendications**

1. Composé qui est un thiazole portant en position 2 un phényle substitué ayant la formule (I) :

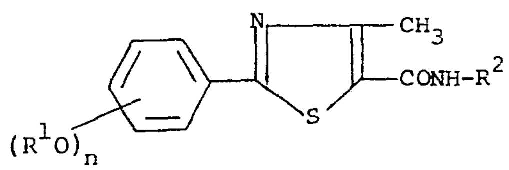

(I)

dans laquelle $R^1$ représente un groupement alkyle possédant de 1 à 3 atomes de carbone ; $R^2$ représente un atome d'hydrogène ou un groupe alkyle possédant de 1 à 3 atomes de carbone ; et n indique un entier de 1 à 3 avec la réserve que lorsque $R^2$ représente un atome d'hydrogène, les positions 3-, 4- et 5- du cycle benzénique ne sont pas simultanément occupées par les groupes méthoxy.

2. Composé suivant la revendication 1, dans lequel $R^1$ est un groupe méthyle.

3. Composé suivant la revendication 1, choisi dans le groupe constitué par le 2-(3,4,5-triméthoxyphényl)-4-méthylthiazole-5-méthylcarboxamide et le 2-(3,4-5-triméthoxyphényl)-4-méthylthiazole-5-n-propylcarboxamide.

4. Composé suivant la revendication 1, qui est choisi dans le groupe constitué par le 2-(3,4-diméthoxyphényl)-4-méthylthiazole-5-carboxamide, le 2-(3,4-diméthoxyphényl)-4-méthylthiazole-5-méthylcarboxamide, et le 2-(3,4-diméthoxyphényl)-4-méthylthiazole-5-n-propylcarboxamide.

5. Composé suivant la revendication 1 qui est choisi dans le groupe constitué par le 2-(2-méthoxyphényl)-4-méthylthiazole-5-carboxamide, le 2-(2-méthoxyphényl)-4-méthylthiazole-5-méthylcarboxamide, le 2-(2-méthoxyphényl)-4-méthylthiazole-5-n-propylcarboxamide, le 2-(4-méthoxyphényl)-4-méthylthiazole-5-carboxamide, le 2-(4-méthoxyphényl)-4-méthylthiazole-5-méthylcarboxamide, et le 2-(4-méthoxyphényl)-4-méthylthiazole-5-n-propylcarboxamide.

6. Procédé de préparation d'un composé de formule (I) tel que décrit dans la revendication 1, ce procédé consistant à effectuer une ammonolyse d'un ester d'alkyle d'un acide 4-méthylthiazole-5-carboxylique portant en position 2 un phényle substitué, l'ester étant représenté par la formule (II) :

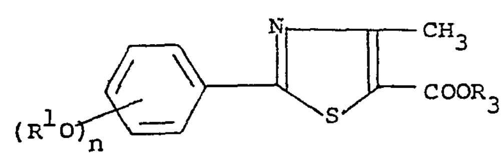

(II)

dans laquelle $R^1$ et n sont tels que définis dans la revendication 1 ; et $R^3$ est un groupe alkyle possédant de 1 à 3 atomes de carbone.

7. Procédé suivant la revendication 6, dans laquelle la réaction est effectuée à une température de − 30 à 150 °C.

8. Procédé suivant la revendication 6 ou 7, dans laquelle l'ester de formule (II) a été obtenu en traitant ensemble à reflux un thioamide d'acide benzoïque substitué de formule :

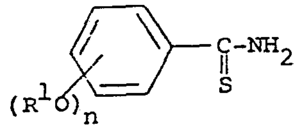

dans laquelle $R^1$ et n sont tels que définis dans la revendication 1, et une alpha-halocétone de formule :

$$CH_3CO\underset{\substack{|\\ X}}{C}H-COOR^3$$

dans laquelle $R^3$ est tel que défini dans la revendication 6 en présence de pentasulfure et dans un solvant organique.

9. Composition pharmaceutique comprenant comme ingrédient actif un composé de formule (I) tel que revendiqué dans l'une quelconque des revendications 1 à 5 ou qui a été préparé par un procédé tel que revendiqué dans l'une quelconque des revendications 6 à 8 en même temps qu'un véhicule pharmaceutiquement acceptable.

10. Composition pharmaceutique convenant pour le traitement des ulcères peptiques du tube digestif, cette composition comprenant sous forme de dosage unitaire une quantité thérapeutiquement efficace d'un composé de formule (I) tel que décrit dans la revendication 1 et un véhicule pharmaceutiquement acceptable.